# EUROPÄISCHE PATENTSCHRIFT

(11) **EP 0 922 181 B1**
(45) Veröffentlichungstag und Bekanntmachung des Hinweises auf die Patenterteilung: **16.03.2005**
(21) Anmeldenummer: 98934962.6
(22) Anmeldetag: 16.06.1998
(51) Int. Cl.: F16M 11/04

(54) **STATIVGEWICHTSAUSGLEICH**
COUNTERWEIGHT FOR STANDS
CONTREPOIDS POUR STATIF

(30) Priorität: 30.06.1997 CH 156697
(43) Veröffentlichungstag der Anmeldung: 16.06.1999
(73) Patentinhaber: Leica Microsystems (Schweiz) AG, 9435 Heerbrugg (CH)
(72) Erfinder: BEES, Bryan, CH-9436 Balgach (CH)
(74) Vertreter: Rosenich, Paul, Dipl.-Ing.
(86) Internationale Anmeldenummer: PCT/EP1998/003614
(87) Internationale Veröffentlichungsnummer: WO 1999/001693

(56) Entgegenhaltungen:
- DE-A- 3 808 327
- US-A- 3 358 957
- US-A- 4 515 333
- US-A- 4 523 732
- US-A- 5 213 293
- US-A- 5 257 998
- US-A- 5 288 043

## Beschreibung

Die Erfindung betrifft ein Stativ, insbesondere ein Stativ für Operationsmikroskope. Neben aufwendigen Konstruktionen mit Gewichtsausgleich für Stative gibt es im Bereich der Operationsmikroskopie auch einfachere Stativaufbauten, die z.B. ohne vertikale Parallelträger auskommen und als Gewichtsausgleich für das In-Balance-Halten eines Operationsmikroskopes eine Gasdruckfeder o.dgl. verwenden. Diesbezüglich wird auf das Patent US-A-5,213,293 der Anmelderin verwiesen. Der Aufbau in einem solchen relativ einfachen Stativ ist so gewählt, dass ein Stativfuss über ausreichende Standfestigkeit verfügt, dass ein Kippen des Statives unter Anwendung herkömmlicher Operationsmikroskope ausgeschlossen ist. Der Stativfuss ist zu diesem Zweck auch relativ schwer ausgebildet, so dass durch ihn eine gewisse Standfestigkeit gewährleistet ist.

Aus der US-A-5,257,998 ist ein Stativ für ein Stereotaxie-System bekannt, das einen Stativfuss und einen daran angeordneten Ständer aufweist. Am Ständer ist ein Tragarm mit einem Ausgleichsgewicht befestigt. Das Ausgleichsgewicht ist als funktionales Gehäuse mit verschiedenen elektrischen Bauteilen ausgestattet. Durch den großen Schwenkbereich des Stativs ist der Stativfuß symmetrisch aufgebaut und damit sehr groß und schwer dimensioniert.

Der Erfindung liegt die Aufgabe zugrunde, eine Modifikation am Stativaufbau vorzunehmen, die es erlaubt, im Bereich der Last, insbesondere des Operationsmikroskopes, grössere Gewichte zu tragen und/oder den Stativfuss schwächer auszubilden.

Bei Stativfüssen ergibt sich dabei ein Problem: Je breiter die Stativfüsse seitlich in den Raum ragen, um so mehr stellen sie eine Behinderung des Personals dar. Man versucht daher die Stativfüsse in ihrer Breitenerstreckung einzuschränken. Eine weitere Aufgabe der vorliegenden Erfindung ist es, dem Wunsch nach leichteren Stativfüssen gerecht zu werden.

Diese Aufgaben werden durch die im kennzeichnenden Teil des Patentanspruchs 1 angegebenen Merkmale gelöst.

Durch die Kombination der Merkmale des Anspruches 1 ergibt sich ein Stativaufbau, der einfach ist, einen praktischen Gewichtsausgleich aufweist und ein reduziertes Kippverhalten aufweist. Weiterbildungen der Erfindung ergeben sich aus den abhängigen Ansprüchen. Zusammen mit der Figurenbeschreibung erschliesst sich dem Fachmann eine neuartige Stativkonstruktion, die einerseits den gestellten Aufgaben, andererseits aber auch den Bedürfnissen nach Aufrüstung herkömmlicher Stative mit Elektronik Rechnung zu tragen.

Die nachfolgende Figurenbeschreibung übergreift alle Figuren. Gleiche Bauteile tragen gleiche Bezugszeichen;
funktionsähnliche - jedoch unterschiedliche - Bauteile tragen gleiche Bezugszeichen mit unterschiedlichen Indizes.

Es zeigen dabei:
- Fig. 1: eine Variante in Seitenansicht, bei der der Tragarm um eine Schwenkachse drehbar ist;
- Fig. 2: eine Aufsicht auf den Aufbau gemäss Fig. 1;
- Fig. 3: eine Variante dazu in Seitenansicht; und
- Fig. 4: einen Schnitt durch Fig. 3 entlang der Linie IV-IV.

Nicht in den Figuren eingetragen sind besondere Stützmechanismen wie Gasdruckfedern o.dgl., wie sie z.B. aus der erwähnten US-A-5,213,293 bekannt sind. Der erfindungsgemässe Aufbau ist jedoch nicht auf solche Ausbildungen eingeschränkt.

Das erfindungsgemässe Stativ verfügt über einen Ständer 4, der einen Tragarm 3 trägt. Als Gewichtsausgleich für ein an einem Schwenkträger montiertes Gewicht, beispielsweise ein Operationsmikroskop 1, dient ein Schaltschrank 5, der mit dem Ständer 4 fest verbunden ist. In dem Schaltschrank 5 befinden sich beispielsweise Elektronik-Baugruppen, Stromversorgungsgeräte, allfällige Einrichtungen für Beleuchtung usw. und bei Bedarf zusätzliche Gewichtseinlagen 14.

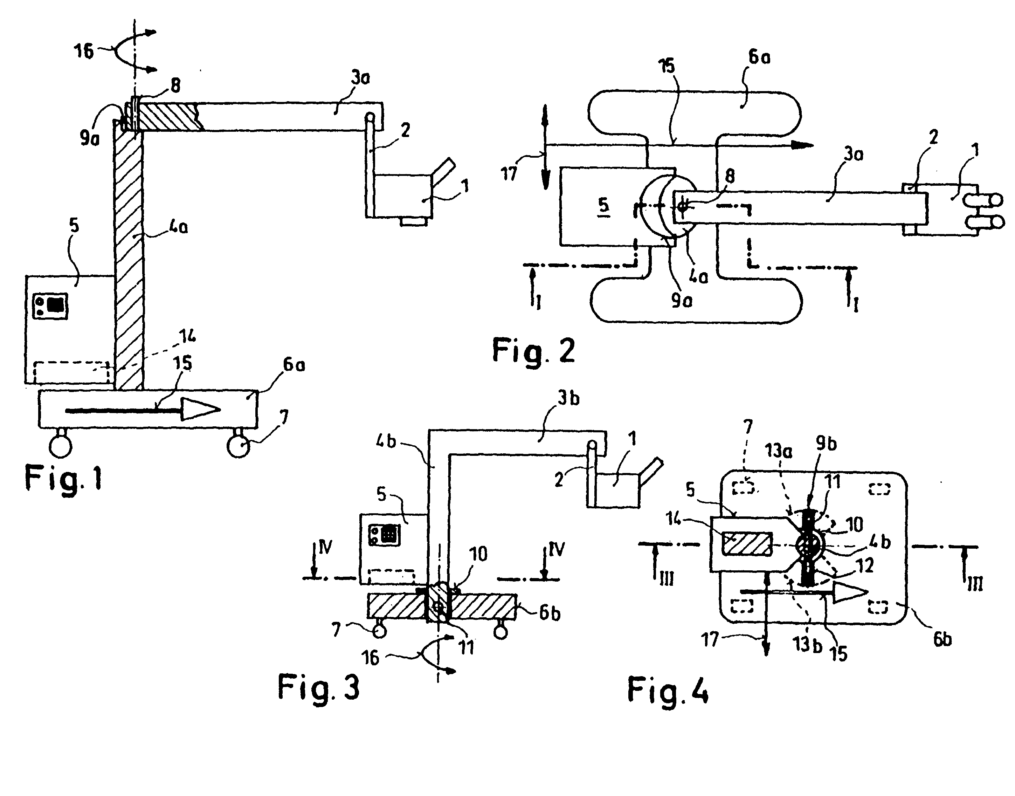

Fig. 1 zeigt eine Ausführungsform, bei der der Tragarm 3a um eine Schwenkachse 8 am Ständer 4a drehbar ist, vgl. den kreisförmigen Doppelpfeil 16. Ein Anschlag 9a begrenzt die seitliche Schwenkbarkeit des Tragarmes 3a in beiden Richtungen. Dadurch wird erreicht, dass der Tragarm 3a nicht in eine Lage schwenken kann, die quer zur bevorzugten Stützrichtung 15 des Stativfusses 6a erstreckt ist. Der Stativfuss 6a ist konstruktiv so ausgelegt, dass er quer zu seiner bevorzugten Stützrichtung 15 eine vergleichsweise geringere Kippsicherheit bietet. Diese Ausführungsform ist geschnitten nach dem Schnitt I-I in Fig. 2.

Fig. 2 verdeutlicht in der Aufsicht den Aufbau gemäss Fig. 1. In dieser Figur wird deutlich, dass der Stativfuss als H-Profil ausgebildet ist und somit in der Stützrichtung (15) eine wesentlich grössere Ausdehnung aufweist als in der dazu senkrechten (Quer-)Richtung (17). Gegenüber einem Quadrat oder einem Kreis wird so ein geringeres Stativfuss-Gewicht bei uneingeschränkter Kippsicherheit in der Stützrichtung 15 erreicht.

Die Fig. 3 und 4 zeigen eine Variante dazu, bei der der Tragarm 3b und der Ständer 4b drehstarr miteinander verbunden sind. Fig. 3 zeigt dabei ein Schnitt gem. III-III aus Fig. 4. Eine Schwenkbewegung gemäss dem Doppelpfeil 16 in einer horizontalen Ebene wird bei diesem Aufbau durch die Drehbarkeit des Ständers 4b im Stativfuss 6b gewährleistet. Hierzu steckt der Ständer 4b in einer Bohrung des Stativfusses 6b und ist gegenüber diesem mit einem starr verbundenen Stützring 10 abgestützt. Wenigstens ein Anschlagbolzen 11 gleitet in einer horizontalen Anschlagnut 13 (13a,13b), die die seitliche Schwenkbewegung des Ständers 4b beschränkt. Zur Montage des Ständers 4b im Stativfuss 6b dienen beispielsweise Montageschlitze 12, die ein Versenken des Ständers 4b mit dem bzw. den Anschlagbolzen 11 im Stativfuss 6b ermöglichen.

Bei beiden dargestellten Varianten ist auf eine mögliche Schwenkbewegung des Tragarmes 3a in einer vertikalen Ebene nicht eingegangen. Eine solche Schwenkbewegung bzw. dazu erforderliche konstruktive Massnahmen sind dem Fachmann in vielfältiger Art und Weise bekannt. Beispielhaft wird auf die bereits erwähnte US-A-5,213,293 und auf das nach dieser Schrift gebaute Stativ der Anmelderin verwiesen.

Das Wesen der vorliegenden Erfindung liegt in der Kombination eines starren Ausgleichsgewichtes, das insbesondere funktionale Teile enthält, am Stativständer, zusammen mit einem Anschlag, der die Schwenkbewegung des Tragarmes in einer horizontalen Ebene, vorzugsweise symmetrisch zur bevorzugten Stützrichtung, begrenzt.

### Bezugszeichenliste

- 1: Operationsmikroskop
- 2: Mikroskopträger
- 3,a,b: Tragarm
- 4,a,b: Ständer
- 5: Schaltschrank/Gehäuse
- 6,a,b: Stativfuss
- 7: Laufrolle oder Stützfuss
- 8: Schwenkachse
- 9,a,b: Anschlag
- 10: Stützring
- 11: Anschlagbolzen
- 12: Montageschlitze
- 13,a,b: Anschlagnut
- 14: Gewichtseinlagen
- 15: bevorzugte Stützrichtung
- 16: Doppelpfeil
- 17: Quer-Richtung

## Patentansprüche

1. Stativ mit einem Tragarm (3), einem Ständer (4) und einem Stativfuss (6), wobei am Ständer (4) ein funktionales Gehäuse (5) mit Gegengewichtsfunktion befestigt ist, **dadurch gekennzeichnet, dass** der Stativfuss (6) so dimensioniert ist, dass er eine bevorzugte Stützrichtung (15) mit vergleichsweise besserer Kippsicherheit, bzw. einen ertaubten Stützbereich seitlich der bevorzugten Stützrichtung (15) aufweist, **und dass** die Schwenkbarkeit des Tragarmes (3) in einer horizontalen Ebene durch einen Anschlag (9) so begrenzt ist, dass der Tragarm (3) nicht in eine Lage quer zur bevorzugten Stützrichtung bzw. nicht ausserhalb des erlaubten Stützbereiches schwenken kann, **und dass** das Gehäuse (5) starr mit dem Ständer (4) verbunden ist.

2. Stativ nach Anspruch 1, **dadurch gekennzeichnet, dass** der Tragarm (3) und der Ständer (4) schwenkfrei miteinander verbunden sind und dass der Anschlag (9b) zur Schwenksicherung im Stativfuss (6) angeordnet ist.

3. Stativ nach Anspruch 1, **dadurch gekennzeichnet, dass** der Anschlag (9a) zur Schwenksicherung zwischen Tragarm (3a) und Ständer (4a) angeordnet ist.

4. Stativ nach einem der vorhergehenden Ansprüche, **dadurch gekennzeichnet, dass** das Gehäuse (5) eine elektronische Steuerung und/oder eine Stromversorgung und/oder Stromwandlereinheiten für den Betrieb des Stativs und/oder des Mikroskops und/oder der Beleuchtung aufweist.

5. Stativ nach einem der vorhergehenden Ansprüche, **dadurch gekennzeichnet, dass** in dem Gehäuse (5) zusätzliche Ausgleichsgewichte angeordnet sind.

6. Stativ nach einem der vorhergehenden Ansprüche, **dadurch gekennzeichnet, dass** der Tragarm (3) federgestützt ist.

7. Stativ nach einem der vorhergehenden Ansprüche, **dadurch gekennzeichnet, dass** der Schwenkbereich symmetrisch zur bevorzugten Stützrichtung verläuft.

8. Stativ nach einem der vorhergehenden Ansprüche, **dadurch gekennzeichnet, dass** der Stativfuss (6) in der bevorzugten Stützrichtung (15) eine wesentlich grössere Ausdehnung aufweist als in der dazu senkrechten (Quer-)Richtung (17).

9. Stativ nach einem der vorhergehenden Ansprüche, **dadurch gekennzeichnet, dass** das Gehäuse (5) oder Gehäuseteile auf dem Stativfuss abgestützt ist (sind).

10. Verwendung eines Stativs nach mindestens einem der vorhergehenden Ansprüche als Träger und Positionierer für ein Operationsmikroskop.

## Claims

1. Stand comprising a support arm (3), a post (4) and a stand base (6), a functional housing (5) having a counterweight function being fastened to the post (4), **characterized in that** the stand base (6) is dimensioned so that it has a preferred support direction (15) with comparatively better security with regard to tilting or a permitted support region to the side of the preferred support direction (15), and **in that** the pivotability of the support arm (3) in a horizontal plane is limited by a stop (9) so that the support arm (3) cannot pivot into a position transverse to the preferred support direction and cannot pivot outside the allowed support range, and **in that** the housing (5) is rigidly connected to the post (4).

2. Stand according to Claim 1, **characterized in that** the support arm (3) and the post (4) are nonpivotably connected to one another, and **in that** the stop (9b) for preventing pivoting is arranged in the stand base (6).

3. Stand according to Claim 1, **characterized in that** the stop (9a) for preventing pivoting is arranged between support arm (3a) and post (4a).

4. Stand according to any of the preceding Claims, **characterized in that** the housing (5) has an electronic control and/or a power supply and/or current transformer units for operation of the stand and/or of the microscope and/or of the lighting.

5. Stand according to any of the preceding Claims, **characterized in that** additional counterweights are arranged in the housing (5).

6. Stand according to any of the preceding Claims, **characterized in that** the support arm (3) is spring-supported.

7. Stand according to any of the preceding Claims, **characterized in that** the pivot range is symmetrical relative to the preferred support direction.

8. Stand according to any of the preceding Claims, **characterized in that** the stand base (6) has a substantially greater dimension in the preferred support direction (15) than in the (transverse) direction (17) perpendicular thereto.

9. Stand according to any of the preceding Claims, **characterized in that** the housing (5) or housing parts is or are supported on the stand base.

10. Use of a stand according to at least one of the preceding Claims as a support and positioner for a surgical microscope.

## Revendications

1. Statif comprenant un avant-bras (3), un montant (4) et un pied de statif (6), un boîtier fonctionnel (5) étant monté au montant (4), qui a la fonction d'un contrepoids, **caractérisé en ce que** le pied de statif (6) est dimensionné de manière qu'il comprend une direction d'appui préférée (15) conférant, en comparaison, une stabilité au déversement améliorée et une zone d'appui permise à côté de la direction d'appui préférée (15), et que le basculement de l'avant-bras (3) est limité dans un plan horizontal par un arrêt (9) de manière que l'avant-bras (3) ne peut pas basculer dans une position transversale à la direction d'appui préférée ou 'hors de la zone d'appui permise, et que le boîtier (5) est relié au montant (4) d'une façon rigide.

2. Statif selon la revendication 1, **caractérisé en ce que** l'avant-bras (3) et le montant (4) sont reliés l'un à l'autre sans basculement, et que l'arrêt (9b) pour bloquer le basculement est disposé dans le pied de statif (6).

3. Statif selon la revendication 1, **caractérisé en ce que** l'arrêt (9a) pour bloquer le basculement est disposé entre l'avant-bras (3a) et le montant (4a).

4. Statif selon une quelconque des revendications précédentes, **caractérisé en ce que** le boîtier (5) comprend un asservissement électronique et/ou une alimentation en courant et/ou des unités de transformation du courant pour le service du statif et/ou du microscope et/ou de l'illumination.

5. Statif selon une quelconque des revendications précédentes, **caractérisé en ce que** des contrepoids supplémentaires sont disposé dans le boîtier (5).

6. Statif selon une quelconque des revendications précédentes, **caractérisé en ce que** l'avant-bras (3) est suspendu par ressort.

7. Statif selon une quelconque des revendications précédentes, **caractérisé en ce que** la zone de basculement s'étend symétriquement à la direction d'appui préférée.

8. Statif selon une quelconque des revendications précédentes, **caractérisé en ce que** le pied de statif (6) a une dimension essentiellement plus grande dans la direction d'appui préférée (15), que dans la direction (transversale) (17) perpendiculaire à celle-ci.

9. Statif selon une quelconque des revendications précédentes, **caractérisé en ce que** le boîtier (5) ou des parties de boîtier s'appuie (s'appuient) au pied de statif.

10. L'emploi d'un statif selon au moins une des revendications précédentes comme support et dispositif de positionnement pour un microscope d'opération.
